# EUROPÄISCHE PATENTSCHRIFT

(11) **EP 1 438 032 B1**
(45) Veröffentlichungstag und Bekanntmachung des Hinweises auf die Patenterteilung: **11.06.2008**
(21) Anmeldenummer: 02767036.3
(22) Anmeldetag: 21.10.2002
(51) Int. Cl.: A61K 9/48

(54) **HERSTELLUNG VON FORMKÖRPERN AUF BASIS VON STÄRKE-GEL**
PRODUCTION OF STARCH-GEL-BASED SHAPED BODIES
FABRICATION DE CORPS MOULES A BASE DE GEL D'AMIDON

(30) Priorität: 23.10.2001 DE 10152125; 06.05.2002 DE 10220264
(43) Veröffentlichungstag der Anmeldung: 21.07.2004
(73) Patentinhaber: InnoGEL AG, 6331 Hünenberg (CH)
(72) Erfinder: MÜLLER, Rolf, CH-8055 Zürich (CH)
(74) Vertreter: Frommhold, Joachim
(86) Internationale Anmeldenummer: PCT/CH2002/000572
(87) Internationale Veröffentlichungsnummer: WO 2003/035044

(56) Entgegenhaltungen:
- EP-A- 1 103 254
- EP-A- 1 258 242
- WO-A-01/85836
- WO-A-02/38132
- WO-A-99/02600

## Beschreibung

Die vorliegende Erfindung betrifft ein Verfahren zur Herstellung von Formkörpern, insbesondere von Wirkstoffen enthaltenden Gelatine-freien Weichkapseln auf Basis von Stärke-Gel.

### Stand der Technik

Entsprechend dem Stand der Technik werden Wirkstoffe enthaltende Formkörper wie Weichkapseln heute noch vorwiegend auf Basis von Gelatine hergestellt. Ein Ersatz von Gelatine für diese Anwendung ist aufgrund der BSE-Problematik jedoch dringlich geworden. Weitere Nachteile der Gelatine sind deren tierischer Ursprung und daher die Nicht-Akzeptanz von Gelatine durch Vegetarier, Veganer, Juden (nicht kosher) und Mohammedaner (Schweine-Gelatine).

An einen Ersatz von Gelatine für diese Anwendung werden primär folgende Anforderungen gestellt: um für die Herstellung von Weichkapseln, insbesondere mittels des Rotary-Die Verfahrens eingesetzt zu werden, muss ein Gelatine-Ersatz zu Filmen geformt werden können, die mindestens eine Dehnung von 100% und eine Festigkeit von mindestens 2MPa bei den Verarbeitungsbedingungen aufweisen, die mit sich selbst verschweissbar sind bei Temperaturen unter 100°C, vorzugsweise bei möglichst tiefen Temperaturen, und sich im Magen auflösen oder zerfallen, damit darin enthaltene Wirkstoffe freigesetzt werden. Ausserdem muss die geformte und verschweisste Kapsel gute Barriereeigenschaften bezüglich der Inhaltsstoffe und eine gute Lagerstabilität aufweisen, d.h. bei Temperaturen im Bereich von 10 bis 40°C und bei Luftfeuchtigkeiten im Bereich von 10 bis 90% möglichst konstante Eigenschaften zeigen. Zusätzlich sollten die die Weichkapselhülle bildenden Stoffe bezüglich der Gelatine preislich konkurrenzfähig sein.

Bisherige Alternativen zur Gelatine sind entwickelt worden, sie haben jedoch verschiedene Nachteile:

US 5'342'626 beschreibt die Herstellung von Weichkapseln mittels des Rotary-Die Verfahrens, wobei die Kapselhülle aus Carrageenan, Mannan-Gums, Gellan bzw. aus Mischungen dieser pflanzlichen Polysaccharide besteht. Die mechanischen Eigenschaften solcher Kapseln sind jedoch unzureichend und die verwendeten Polysaccharide ausserdem gegenüber Gelatine teilweise deutlich teuer.

EP 0'397'819 beschreibt ein Verfahren zur Herstellung von Thermoplastischer Stärke mit nur geringem kristallinen Anteil. Die Bruchdehnung entsprechender Filme ist jedoch deutlich geringer als benötigt und die Verschweissbarkeit problematisch. Ausserdem ist eine ausgeprägte Abhängigkeit der Kapseleigenschaften von der Luftfeuchtigkeit gegeben.

EP 0'542'155 beschreibt Thermoplastische Stärke und Cellulosederivate enthaltende Mischungen, die wiederum ungenügende mechanische Eigenschaften aufweisen und infolge des hohen Preises der verwendeten Cellulosederivate auch in dieser Hinsicht nur beschränkt konkurrenzfähig sind.

In WO 01/37817 A1 wird ein Verfahren zur Herstellung von Weichkapseln auf Basis von Thermoplastischer (TPS) Stärke mit hohem Weichmachergehalt beschrieben. Allerdings wirken sich die typischen problematischen Eigenschaften von TPS, die auch einem Durchbruch von TPS als biologisch abbaubarem Kunststoff im Wege standen, namentlich deren inhärente Sprödigkeit und nachteiliges Sorptionsverhalten, wodurch die mechanischen Eigenschaften stark von der Luftfeuchtigkeit abhängig sind, auch bezüglich einer Verwendung von TPS als Gelatine-Ersatz im Bereich der Weichkapseln nachteilig aus. Dies hat zur Folge, dass die Lagerstabilität entsprechender Kapseln ungenügend ist und diese selbst bei hohen Weichmachergehalten, insbesondere bei tiefen Temperaturen und niederer Luftfeuchtigkeit, zur Versprödung neigen und zerbrechlich werden.

In WO 02/38132 A2 wird die Herstellung von Weichkapseln beschrieben, wobei eine Lösung enthaltend Stärke und mindestens eine Stärkekomponente mit einem gegenüber nativer Stärke verringerten Verzweigungsgrad hergestellt und anschliessend geliert wird. Die Gelierung basiert dabei primär auf der Stärke mit verringertem Verzweigungsgrad. Dieses Stärke-Gel wird nach erfolgter Gelierung über ein Formgebungsverfahren zu einem Film geformt und dieser dann anstelle von Gelatine-Film für die Verkapselung mittels des Rotary-Die Verfahrens verarbeitet, wobei jeweils die zwei Kapselhälften gebildet, mit Wirkstoff gefüllt und verschweisst werden. Nach erfolgter Verkapselung werden die Kapseln getrocknet. Diese Lösung hat jedoch folgende Nachteile: 1) Da die Stärke mit verringertem Verzweigungsgrad zusammen mit anderen Stärken gelöst wird, wobei ein grosser Teil des Lösungsmittels von diesen Stärken beansprucht wird, steht der Komponente mit verringertem Verzweigungsgrad ein entsprechend reduzierter Lösungsmittelanteil zur Verfügung, wodurch deren Löslichkeit massiv eingeschränkt wird. Bei den in dieser Offenlegungsschrift angegebenen Bedingungen können somit nur schwach entwickelte Gele erhalten werden. Um die Löslichkeit der Stärke mit verringertem Verzweigungsgrad und damit deren anschliessende Gelbildung zu verbessern, müssen sehr hohe Lösungsmittelkonzentrationen angewandt werden, wodurch jedoch die Festigkeit der dann gebildeten Gele für das Rotary-Die Verfahren nicht ausreichend ist. 2) Nachdem die Gele gebildet worden sind, liegt infolge der entwickelten Netzwerkstruktur eine eingeschränkte Dehnbarkeit vor. Während für das Rotary-Die Verfahren Dehnungen von mindestens 100% gefordert werden, können mit Stärke-Gelen, die entsprechend dem Stand der Technik allgemein als spröde gelten, jedoch nur geringe Dehnungen im Bereich von 10 bis höchstens 40% erhalten werden, wodurch die Herstellung von gebräuchlichen Weichkapseln wie beispielsweise Oblong-Kapseln nicht möglich ist. Die eingeschränkte Dehnbarkeit der Gel-Filme lässt nur sehr "flache" Kapseln mit geringem innerem Volumen zu, welche vom Markt nicht akzeptiert werden. 3) Während weichgemachte TPS eine Verschweissung zulässt, ist dies bei Stärke-Gelen nur sehr beschränkt der Fall, insbesondere werden dazu Temperaturen von mindestens 130°C (wobei auch bei dieser nicht-praktikablen Temperatur nur ungenügende Verschweissungen erhalten werden können), d.h. eine zumindest teilweise Auflösung der Netzwerkstruktur benötigt, weswegen die Herstellung von verschweissten marktgängigen Weichkapseln auf Basis von Stärke-Gel entsprechend dem vorgeschlagenen Verfahren auch aus diesem Grund nicht wirklich machbar ist. Bezüglich der in der Offenlegungsschrift WO 02/38132 A2 erwähnten weiteren Varianten von Stärke-Gelen sind die erwähnten Probleme sogar noch ausgeprägter.

### Erfindung

Gegenstand der Erfindung sind Verfahren zur Herstellung von Formkörpern auf Basis von Stärke-Gel, danach hergestellte Formkörper, sowie deren Verwendung gemäss den Ansprüchen 1-17.

Aufgrund der Problematik der gegenwärtigen Technologien zur Herstellung von Gelatine-freien Weichkapseln wird ein neues Verfahren gemäss Anspruch 1 vorgeschlagen, wodurch gegenüber anderen vorgeschlagenen Lösungen entscheidende Vorteile erhalten werden können. Vorteilhafte Ausführungsformen sind in den abhängigen Ansprüchen offenbart. Das Verfahren beruht darauf, dass anstelle einer Lösung von Stärke und Stärke mit verringertem Verzweigungsgrad eine vorliegende Stärke, die eine beliebige Stärke sein kann, durch ein thermoplastisches Verfahren plastifiziert, eine netzwerk- oder gelierfähige Stärke oder eine Mischung solcher Stärken separat gelöst, dann der vorliegenden plastizifierten Stärke zugemischt, mit dieser vorzugsweise molekulardispers gemischt wird, wobei als Resultat des Mischvorgangs immer noch eine Schmelze (und kein Gel) vorliegt, die in einem nächsten Schritt zu Filmen oder Bändern geformt werden, welche dann dem Rotary-Die Verfahren zugeführt werden können. Von grundlegender Bedeutung ist dabei, dass die Gel- oder Netzwerkbildung nach der Film-Herstellung höchstens teilweise vor dem Verkapselungsschritt eingeleitet wird, wodurch die gute Dehnbarkeit von plastifizierter Stärke und deren Verschweissbarkeit erhalten bleibt. Die Netzwerkbildung ist zu nennenswerten Anteilen erst nach erfolgter Verkapselung erwünscht und ermöglicht dann folgende Vorteile:

Das Einsetzen der Netzwerkbildung nach erfolgter Verschweissung ermöglicht die für das Rotary-Die Verfahren vorausgesetzte Dehnbarkeit von mindestens 100% wie auch eine Verbesserung der Schweissnaht, da diese durch das Netzwerk verstärkt wird, was einfach dadurch veranschaulicht werden kann, dass die Festigkeit der Schweissnaht nicht nur auf der Verhakung von Stärke-Makromolekülen, wie dies bei der Verschweissung von TPS der Fall ist, sondern zusätzlich auch auf einer Netzwerkbildung beruht, wobei Stärke-Makromoleküle die jeweils den beiden zu verschweissenden Hälften der Weichkapseln angehören, durch Mikrokristallite, welche die elastisch aktiven Vernetzungspunkte des Netzwerks bilden, miteinander verknüpft werden.
2) Ein nach der Verkapselung gebildetes Netzwerk kann als eine elastische "innere Armierung" veranschaulicht werden, wodurch einerseits die Festigkeit der Weichkapseln verbessert wird, andererseits auch deren Zähigkeit (reduzierte Sprödigkeit). Indem eine eingeschränkte Netzwerkbildung bei der Formung der Kapseln zugelassen wird, kann ausserdem auch eine Verbesserung der Schmelzfestigkeit der plastifizierten Stärke und derer Dehnbarkeit erhalten werden. Da Stärke infolge der Steifigkeit der Makromoleküle nur eine beschränkte Verhakung dieser Makromoleküle zulässt, wodurch die Schmelzfestigkeit und auch die Festigkeit der Schweissnaht eingeschränkt wird, ermöglicht eine geringe teilweise Netzwerkbildung eine Verbesserung, indem durch Mikrokristallite gebildete Netzwerkelemente die Funktion der Verhakungspunkte ergänzen, bzw. übernehmen. Darüber hinaus sind elastische Netzwerkelemente auch effektiver als Verhakungspunkte, die insbesondere bei steifen Makromolekülen nur eine relativ lockere Fixierung von Makromolekülen ermöglichen. Entsprechend einem weiteren Gesichtspunkt kann die Situation auch so betrachtet werden, dass durch eine geringe teilweise Netzwerkbildung vor und/oder während der Formung der resultierenden Formkörper bzw. Weichkapseln ein quasi erhöhtes Molekulargewicht erzielt wird, indem verschiedene Makromoleküle durch Netzwerkelemente physikalisch miteinander verknüpft werden. Dies resultiert in einem ausgeprägten strukturviskosen Verhalten und einer erhöhten Schmelzfestigkeit und Schmelzdehnbarkeit. Da durch die Verfahrensparameter und über die Rezeptur die Kinetik der Netzwerkbildung einfach beeinflusst werden kann, ist die Einstellung einer eingeschränkten Netzwerkbildung unproblematisch. Eine Verbesserung der Zähigkeit (oder Verringerung der Sprödigkeit) von Stärke-Netzwerken gegenüber TPS ergibt sich einerseits durch die elastische "innere Armierung", wobei dies jedoch nur für beschränkte Dehnungen bis höchstens 40% gilt, was jedoch für gefertigte Weichkapseln mehr als ausreichend ist, und andererseits durch die Teilkristallinität des durch Mikrokristallite gebildeten Netzwerks, wenn diese Mikrokristallite genügend fein verteilt sind. Je feiner verteilt die durch Mikrokristallite gebildeten elastischen Netzwerkelemente in der vorwiegend amorphen Matrix vorliegen, umso grösser sind die positiven Effekte des Netzwerks bezüglich Festigkeit und Zähigkeit. Eine hochdispersive Verteilung der Netzwerkelemente wird durch einen hohen Dispergierungsgrad der in die vorliegende plastifizierte Stärke eingemischten, netzwerkfähige Stärken enthaltenden Lösung erreicht. Optimal ist eine molekulardisperse Mischung, welche beispielsweise durch hohe Schergeschwindigkeiten beim Mischvorgang erhalten werden kann. Eine nachfolgende Phasenseparation, wodurch die mechanischen Eigenschaften dann wieder verschlechtert würden, kann durch eine geeignete Temperaturführung und durch die Einstellung einer hohen Schmelzviskosität, wodurch der thermodynamisch instabile Zustand einer hochdispersen Mischung eingefroren wird, kinetisch unterdrückt werden. Mittels molekulardisperser Mischung können insbesondere auch einphasige Stärke-Gele und durch Heterokristallisation gebildete Stärke-Gele erhalten werden, die vollständig transparent sind und optimale mechanische Eigenschaften aufweisen.
3) Die kristallinen Anteile zeigen gegenüber dem amorphen Anteil eine reduzierte Wasseraufnahme und resultiert damit für Stärke-Gele gegenüber TPS, die praktisch vollständig amorph ist, ein deutlich günstigeres Sorptionsverhalten, wodurch dem Nachteil der ausgeprägten Sprödigkeit von TPS-Weichkapseln bei niederen Luftfeuchtigkeiten durch Einsatz von Stärke-Gel begegnet werden kann. Besonders ausgeprägt ist dieser vorteilhafte Effekt, wenn die Mikrokristallite im kristallographischen A-Strukturtyp erhalten werden können, da der A-Strukturtyp gegenüber dem üblicherweise sich einstellenden B-Strukturtyp, der bei Raumtemperatur der stabile Strukturtyp ist, nur einen Bruchteil an Wasser aufnehmen kann. Der bei Raumtemperatur metastabile A-Strukturtyp kann durch geeignete Verfahrensparameter, insbesondere durch eine geeignete Temperaturführung oder durch eine nachträgliche Wärmebehandlung erhalten und eingefroren werden.
4) Neben den verschiedenen Vorteilen für die Herstellung von Weichkapseln, welche durch ein sich während dem Formgebungsvorgang höchstens teilweise, vorwiegend nach der Verschweissung bildenden Netzwerks oder Gels erhalten werden können, besteht weiter die Differenzierung gegenüber TPS-Weichkapseln, die sich in Wasser oder im Magensaft auflösen, dass sich Netzwerke in Wasser und im Magen nicht lösen, sondern quellen. Bei den für Weichkapseln verwendeten Wandstärken und Anteilen von netzwerkfähigen Stärken quillt das Stärke-Gel in Wasser oder in Magensaft innerhalb von Minuten und verliert es dabei seine Festigkeit fast vollständig, wodurch die Kapseln leicht zerfallen und daher die Freisetzung der in den Weichkapseln enthaltenen Wirkstoffe unproblematisch ist. Bei höheren Netzwerkdichten, welche beispielsweise durch erhöhte Anteile an netzwerkfähigen Stärken eingestellt werden können, kann der Zerfall von Stärke-Gel Weichkapseln durch gängige Sprengmittel wie Calciumcarbonat verbessert und beschleunigt werden. Von Bedeutung ist das unterschiedliche Zerfallsverhalten im wässrigen Milieu von TPS und Stärke-Gel insofern, dass hierdurch eine eindeutige und einfach zu verifizierende Differenzierung zwischen den zwei verschiedenen Kapseltypen möglich ist. Bei erhöhten Netzwerkdichten, die sich durch das vorgeschlagene Verfahren einfach erhalten lassen, indem der Anteil netzwerkfähiger Stärken erhöht wird, kann der Quellgrad der resultierenden Stärke-Gele bei Bedarf soweit eingeschränkt werden, dass die resultierenden Kapseln auch nach erfolgter Quellung in Wasser oder in Magensaft eine genügende Festigkeit aufweisen, um einen Zerfall zu verhindern. Somit ergibt sich die Möglichkeit einer retardierten Freisetzung der in den Kapseln enthaltenden Wirkstoffe durch Diffusion derselben durch die eingeschränkt gequollene Kapselhülle. Somit können also auf der Basis des vorgeschlagenen Verfahrens zur Herstellung von Weichkapseln auch Controlled-Release-Systeme hergestellt werden.
5) Ein weiterer Vorteil von auf Stärke-Gel basierenden Weichkapseln ist dadurch gegeben, dass vergleichbar mit resistenter Stärke, Stärke-Gel prebiotisch ist. Resistente Stärke werden trotz ihres gegenüber üblichen Stärken mehrfach höheren Preises wegen ihrer prebiotischen Wirkung zunehmend verschiedenen Nahrungsmitteln als Zuschlagstoff zugesetzt. Dieser Effekt ist bei Weichkapseln auf Basis von Stärke-Gel gewissermassen als Zugabe automatisch enthalten. Weiter sind resistente Anteile enthaltende Weichkapseln infolge des gegenüber TPS-Weichkapseln reduzierten Glyceamic-Index auch für Diabetiker besonders vorteilhaft. Darüber hinaus ist zu erwähnen, dass der prebiotische Effekt wie auch die Reduktion des Glyceamic-Index von Stärke-Gel gegenüber resistenter Stärke ausgeprägter ist. Dies ist neben der Abwesenheit von bedenklicher Gelatine in auf Stärke-Gel basierenden Weichkapseln ein weiterer wichtiger Faktor, wodurch die Attraktivität solcher Weichkapseln gesteigert werden kann, umso mehr, als damit nicht nur der Inhalt von Weichkapseln sondern auch die Kapselhülle selbst gesundheitsfördernd ist. Unter den verschiedenen gesundheitsfördernden Wirkungen von resistenter Stärke, insbesondere von resistentem Stärke-Gel, sind die Stimulierung des Immunsystems und die Hemmung von Colon-Krebs besonders aktuell.
6) Durch Zugabe und Einmischen während des Mischvorgangs von vorliegender Stärke und der Lösung von netzwerkfähiger Stärke eines Pulvers von Stärke-Gel, das speziell hinsichtlich der prebiotischen Wirkung optimiert worden ist, kann der prebiotische Effekt von Stärke-Gel-Weichkapselhüllen weiter gesteigert und der Glyceamic-Index weiter reduziert werden. Dabei kann noch ein weiterer Vorteil erhalten werden, resultierend daraus, dass das pulverförmige prebiotische Stärke-Gel eine sehr stark eingeschränkte Wasseraufnahme zeigt, wodurch das Sorptionsverhalten von diese Komponente als zweite Phase enthaltenden Weichkapseln weiter verbessert werden kann. Da diese hochresistente zweite Phase und die Stärke-Gel Matrix stofflich identisch sind, sich nur durch unterschiedliche Netzwerkdichten unterscheiden, ist die Phasenkopplung unproblematisch.
7) Im Vergleich zu WO 01/37817 A1 können infolge der teilkristallinen, bzw. mikrokristallinen Gel-Struktur Weichmacher wie beispielsweise Wasser, Sorbitol, Maltitol oder Mannitol in geringeren Anteilen eingesetzt werden, sodass das Sorptionsverhalten und damit die Produkteigenschaften unter wechselnden Luftfeuchtigkeiten auch aufgrund dieses Effektes verbessert werden können.

Insgesamt ergeben sich gegenüber bestehenden Lösungen im Bereich von Gelatine-freien Weichkapsel mit der vorgeschlagenen Erfindung eine ganze Reihe von Vorteilen bezüglich der mechanischen Eigenschaften, des Verschweiss- und des Sorptionsverhalten, bezüglich eines grösseren Verfahrensspielraums, wodurch eine ganze Reihe von Weichkapseln mit spezifisch optimierten Eigenschaften hergestellt werden können sowie auch bezüglich neuer Eigenschaften, die im Bereich von Weichkapseln bisher noch nicht in Erwägung gezogen worden sind, namentlich die auf Stärke-Gel beruhende prebiotischen Wirkung und deren im Vergleich mit Stärke oder TPS reduzierter Glyceamic-Index, wodurch die neue Technologie vorzügliche Markt- und Marketing-Möglichkeiten eröffnet. Dies ist von umso grösserer Bedeutung, als die alte Gelatine-Technologie neben der Gelatine-Problematik auch aufgrund von Billig-Nachahmern deutlich an Attraktivität verloren hat.

Das vorgeschlagene Verfahren kann vereinfacht dadurch charakterisiert werden, dass eine vorliegende Stärke oder eine Mischung vorliegender Stärken, ganz oder teilweise plastifiziert, bei vergleichsweise niederem Weichmachergehalt mit mindestens einer ganz oder teilweise gelösten netzwerkfähigen Stärke oder mit mindestens einer ganz oder teilweise gelösten Mischung von verschiedenen netzwerkfähigen Stärken mit vergleichsweise hohem Weichmachergehalt molekulardispers gemischt wird. Dies ist eine wesentliche Voraussetzung insbesondere zur Bildung von einphasigen Stärke-Gelen. Wichtige Verfahrensmassnahmen sind dabei eine Überhitzung der netzwerkfähigen Stärke, und gegebenenfalls eine nachfolgende Unterkühlung vor dem Mischvorgang mit der vorliegenden Stärke. Durch diese beiden Massnahmen kann die Temperatur, bei der anschliessend die Netzwerkbildung einsetzt, auf den erwünschten Bereich eingestellt werden, insbesondere ist es möglich, den Beginn der Netzwerkbildung so zu programmieren, dass beim Schritt der Herstellung der resultierenden Formkörper bzw. Kapseln, eine teilweise Netzwerkbildung vorliegt, wodurch die Schmelzfestigkeit und Schmelzdehnbarkeit verbessert, die Verschweissung jedoch noch nicht beeinträchtigt wird. Die Ausbildung der einphasigen Netzwerkstruktur wird durch die Auswahl der Komponenten, wobei die Molekulargewichte von primärer Bedeutung sind, sowie durch die kinetische Kontrolle des Gelierungsvorgangs mittels der entsprechenden Verfahrensparameter ermöglicht. Die Mischung kann dann zu Filmen geformt werden, die dem Rotary-Die Prozess zugeführt werden.

### Vorliegende Stärke

Als vorliegende Stärke kann irgendeine Stärke oder irgendein Mehl, wie auch Mischungen verschiedener Stärken und/oder Mehlen eingesetzt werden. Die vorliegenden Stärken können gelierfähig sein oder auch nicht. Die vorliegende Stärke in einem beliebigen Zustand, physikalisch und/oder chemisch modifiziert dem Verfahren zugeführt werden.

Beispiele für in Frage kommende vorliegende Stärken oder Mehle sind folgenden Ursprungs: Getreide wie Mais, Reis, Weizen, Roggen, Gerste, Hirse, Hafer, Dinkel etc.; Wurzeln und Knollen wie Kartoffel, Süsskartoffel, Tapioka (Cassava), Maranta (Arrowroot) etc.; Hülsenfrüchte und Samen wie Bohnen, Erbsen, Mungo, Lotus etc.. Daneben kommen auch Stärken und Mehle anderen Ursprungs in Frage wie beispielsweise Sago, Yarns etc.. Ausserdem kann auch Glycogen eingesetzt werden.

Die Stärken können durch Züchtung oder gentechnische Methoden verändert worden sein wie beispielsweise Waxy-Mais, Waxy-Reis, Waxy-Kartoffel, hochamylosehaltiger Mais, indica Reis, japonica Reis et., sie können durch chemische Verfahren verändert worden sein wie beispielsweise durch Säure-Konvertierung, Pyrokonvertierung, Vernetzung, Acetylierung, Hydroxyethylierung, Hydroxypropylierung, Phosphorylierung, Graft-Reaktionen, Reaktionen mit Amylasen et., sie können durch physikalische Verfahren verändert worden sein wie beispielsweise durch Gelatinisierung (teilweise bis vollständig), Plastifizierung, Inhibierung et., oder sie können durch eine Kombination von Züchtung, genetische Methoden, chemische und physikalische Verfahren verändert worden sein.

Beispiele für veränderte Stärken sind dünnkochende Stärken, kaltwasserlösliche Stärken, pregelatinisierte Stärken, hydroxypropylierte Stärken, Dextrine, Maltodextrine, limit-Dextrine, Oligosaccharide, kationische Stärken, Stärkeether, durch Fraktionierung erhaltene Stärken.

Von besonderem Interesse sind vorliegende Stärken, deren Amylopektin-Fraktion eine mittlere Kettenlänge CL von mindestens 20, vorzugsweise von mindestens 22, noch bevorzugter von mindestens 24, am bevorzugtesten von mindestens 26 aufweisen.

Weiter sind von besonderem Interesse vorliegende Stärken, deren Amylopektin-Fraktion einen Blue-Value (BV) von mindestens 0.10, vorzugsweise von mindestens 0.13, noch bevorzugter von mindestens 0.16, am bevorzugtesten von mindestens 0.18 aufweisen.

Ebenfalls sind von besonderem Interesse vorliegende Stärken, deren Amylopektin-Fraktion eine Jod-Affinität (IA) in g/100g von mindestens 0.4, vorzugsweise von mindestens 0.6, noch bevorzugter von mindestens 0.8, am bevorzugtesten von mindestens 1.0 aufweisen.

Bezüglich des Molekulargewichts M_{w} (Gewichtsmittel) von vorliegenden Stärken sind von besonderem Interesse Stärken mit einem Gewichtsmittel von mehr als 10'000g/mol, vorzugsweise von mehr als 50'000g/mol, noch bevorzugter von mehr als 100'000g/mol, am bevorzugtesten von mehr als 500'000g/mol.

### Netzwerkfähige Stärken

Netzwerkfähige Stärken lassen sich auf folgende Arten definieren.
1. Entsprechend einer ersten Definition können dies Stärken oder Mehle sein, die unter geeigneten Bedingungen Gele bilden können. Davon ausgenommen sind Gele wie reine Amylopektin-Gele, die sehr lange Gelierungszeiten (Tage bis Wochen) benötigen und dann nur sehr schwache Gele bilden. Netzwerkfähige Stärken können nativ oder physikalisch und/oder chemisch modifiziert worden sein.
1A. Eine Gruppe von Stärken, die dieser Anforderung genügen, sind native oder modifizierte Stärken mit einem Amylosegehalt von mindestens 10%, vorzugsweise von mindestens 20%, noch bevorzugter von mindestens 30%, am bevorzugtesten von mindestens 50%. Besonders geeignet sind beispielsweise hochamylosehaltige Stärken, insbesondere hochamylosehaltige Maisstärken, die einen Amylosegehalt bis nahezu 100% aufweisen können, Erbsenstärken mit Amylosegehalten von mehr als 25% oder Amylosen beliebigen Ursprungs.
1B. Eine weitere Gruppe von netzwerkfähigen Stärken, kann durch chemischen und/oder enzymatischen Abbau, insbesondere durch Entzweigung erhalten werden. Für den enzymatischen Abbau können Amylasen, wie alpha-Amylase, beta-Amylase, Glucoamylase, alpha-Glucosidase, exo-alpha-Glucanase, Cyclomaltodextrin, Glucanotransferase, Pullulanase, Isoamylase, Amylo-1,6-Glucosidase oder eine Kombination dieser Amylasen eingesetzt werden. Als Ausgangsstoffe für den Abbau werden dabei insbesondere Stärken der vorgenannten Gruppe von Stärken eingesetzt. Ein Beispiel von chemischem, nicht-enzymatischem Abbau von Stärken, ist die Hydrolyse mittels Säuren wie etwa Salzsäure.
2. Eine weitere Definition netzwerkfähiger Stärken bezieht sich auf den Verzweigungsgrad Q_{b}, wobei der Verzweigungsgrad kleiner ist als 0.01, vorzugsweise kleiner als 0.005, noch bevorzugter kleiner als 0.002 am bevorzugtesten kleiner als 0.001, insbesondere kleiner als 0.0001.
3. Ausserdem werden als netzwerkfähige Stärken auch vorwiegend lineare Stärken bezeichnet, die nach erfolgter Lösung kristallisieren können, in Abwesenheit weiterer Stärken jedoch keine Gele, sondern Dispersionen von Kristalliten bilden. Solche Stärken haben mittlere Polymerisationsgrade DP von typischerweise weniger als 100, sie können jedoch in Anwesenheit von Stärken, die sowohl nicht netzwerkfähig, als auch netzwerkfähig sein können, durch Heterokristallisation Gele bilden. Bezüglich dieses Typs von netzwerkfähigen Stärken sind Stärken von Interesse, die eine mittlere Kettenlänge CL oder einen mittleren Polymerisationsgrad von mindestens 10, vorzugsweise von mindestens 20, noch bevorzugter von mindestens 30, am bevorzugtesten von mindestens 50 aufweisen. Im Falle von Stärken kann eine solche netzwerkfähige Stärke beispielsweise ein entzweigtes Maltodextrin sein, das für sich keine Gele bilden kann, jedoch mit einem Amylopektin Gele bildet, die Amylose-Gelen vergleichbar sind.
4. Netzwerkfähige Stärken können andererseits dadurch charakterisiert werden, dass die Makromoleküle lineare Anteile enthalten, wobei diese linearen Anteile Haupt- oder Seitenketten sein können mit mittleren Polymerisationsgraden DP von mehr als 30, vorzugsweise mehr als 50, am bevorzugtesten mehr als 80, insbesondere von mehr als 100, am insbesondersten von mehr als 140.
5. Ausserdem kann eine weitere Gruppe von netzwerkfähigen Stärken durch Fraktionierung von Amylose-Amylopektin-Mischungen erhalten werden, beispielsweise durch Fraktionierung mittels differentieller Alkoholfällung, wobei die Amylose- und die intermediat Fraktion als netzwerkfähige Stärke eingesetzt werden kann.

Erfindungsgemäss werden als netzwerkfähige Stärke Stärken bezeichnet, welche mindestens eine der Bedingungen 1 - 5 erfüllen. Als netzwerkfähige Stärke werden auch Mischungen bezeichnet, wobei die Komponenten und/oder die Mischung mindestens eine der obigen Bedingungen erfüllen.

Es wird darauf hingewiesen, dass in bestimmten Fällen vorliegende Stärke und netzwerkfähige Stärke stofflich identisch sein können, da im Prinzip jede netzwerkfähige Stärke auch als vorliegende Stärke verwendet werden kann. Der Unterschied zwischen vorliegender Stärke und netzwerkfähiger Stärke ist daher nicht in allen Fällen stofflicher Art, vielmehr müssen die Begriffe auch in Zusammenhang mit dem Verfahren definiert werden. Netzwerkfähige Stärken werden in einer Weise behandelt, dass deren Potential zur Bildung von Netzwerken optimal freigesetzt wird, während dies bei vorliegenden Stärken ohne geeigneten Löse- und Unterkühlungsvorgang nicht der Fall sein muss.

### Verfahren

### 1. Lösen und gegebenenfalls Unterkühlen netzwerkfähiger Stärken

Erst durch geeignetes Lösen von netzwerkfähigen Stärken wird ihr Potential zur Bildung von Netzwerken freigesetzt. Durch Plastifizierung, wie dies beispielsweise bei der Herstellung von Thermoplastischer Stärke üblich ist, ist dies höchstens teilweise gewährleistet oder sind bei tiefer Weichmacherkonzentration sehr hohe Temperaturen notwendig, die dann zu einem starken thermischen Abbau führen. Der Lösevorgang von netzwerkfähigen Stärken ist ein vielstufiger und komplexer Vorgang. Der Lösevorgang erstreckt sich üblicherweise über einen Temperaturbereich von einigen °C, wobei sukzessive Ordnungsstrukturen aufgelöst werden, bis eine vollständige Lösung erfolgt ist. Der Temperaturbereich zeigt ausserdem eine starke Abhängigkeit von der Konzentration. Der Lösevorgang ist weiterhin auch abhängig von einer mechanischen Beanspruchung durch Scherung, wodurch das Lösen bei tieferer Temperatur erfolgen kann, sowie vom Druck, der Lösungszeit, der Aufheizgeschwindigkeit und dem pH.

Allerdings wird eine Überhitzung der Lösung bevorzugt, wobei eine vollständige Lösung, somit eine Standardisierung erhalten wird. Unter Überhitzung wird der Vorgang verstanden, wobei eine Temperatur angewendet wird, die höher liegt als die Lösungstemperatur. Die für die Netzwerkbildung wirksamen Keime können danach in grösserer Anzahl und Effektivität durch eine definierte Unterkühlung erhalten werden, wodurch sich sehr fein strukturierte Netzwerke mit entsprechend guten mechanischen Eigenschaften herstellen lassen, insbesondere auch einphasige Gele. Die verschiedenen Parameter des Löse- und Unterkühlungsvorgangs sind deshalb für die Struktur und Eigenschaften der nach dem Mischvorgang mit vorliegenden Stärken sich ergebenen Gele von zentraler Bedeutung.

Verschiedene netzwerkfähige Stärken können zusammen gelöst, unterkühlt und dann vorliegenden Stärken zugemischt werden. Da jedoch verschiedene netzwerkfähige Stärken eine unterschiedliche Löse- und Keimbildungscharakteristik aufweisen, ist es oft sinnvoll, sie getrennt aufzubereiten und getrennt dem Mischvorgang zuzuführen.

Da netzwerkfähige Stärken Lipide und Proteine enthalten können, welche mit den linearen Anteilen der netzwerkfähigen Stärken Komplexe bilden und damit diese linearen Anteile für die Netzwerkbildung nicht mehr zur Verfügung stehen, ist es bei höheren Lipid- und Proteinanteilen angezeigt, diese Stoffe vorgängig durch Extraktion zu entfernen. Sie können jedoch auch nach dem Lösevorgang, bei der nachfolgenden Unterkühlung, wobei sie aus der Lösung ausfallen, durch Filtration aus dem Prozess entfernt werden. Bevorzugt werden netzwerkfähige Stärken stammend von Wurzel- oder Knollen-Stärken eingesetzt, welche nur verschwindend kleine Anteile an Proteinen aufweisen.

Die Parameter des Löse- und Unterkühlungsvorgangs sind wie folgt:
1. Der Weichmachergehalt WM_{d} in Gew.% der netzwerkfähigen Stärken in Schritt d) und e) liegt im Bereich 40% - 99%, vorzugsweise im Bereich 45% - 97%, noch bevorzugter im Bereich 50% - 95%, am bevorzugtesten im Bereich 60% - 92%.
2. Der Druck p während dem Überführen in Schritt d) und e) ist identisch mit dem Wasserdampfdruck p_{w}(T) bei der jeweiligen Temperatur, vorzugsweise maximal 2 p_{w}(T), noch bevorzugter maximal 5 p_{w}(T), insbesondere maximal 10 p_{w}(T), am bevorzugtesten maximal 100 p_{w}(T).
3. Die Überhitzungs-Temperatur T_{LÜ} in Schritt d) beträgt mindestens 120°C, vorzugsweise mindestens 140°C, noch bevorzugter mindestens 160°C, insbesondere mindestens 180°C, am bevorzugtesten mindestens 200°C. Sie kann maximal bis 260°C betragen, wobei solch hohe Temperaturen nur für sehr kurze Zeiten zur Anwendung kommen können. Hohe Temperaturen T_{LÜ} wirken sich stabilisierend auf die Lösung aus, d.h. je höher T_{LÜ}, bei umso tieferer Temperatur bleibt die Lösung danach noch stabil, wodurch der Verfahrensspielraum vergrössert wird. Insbesondere sind hohe Temperaturen T_{LÜ} von Bedeutung, um den Beginn der Netzwerkbildung nach erfolgter Mischung der Lösung mit vorliegender Stärke zu steuern. Je höher T_{LÜ}, bei umso tieferer Temperatur setzt dann die Netzwerkbildung ein.
4. Die Zeitdauer deltat _{d} des Überführens in Schritt d) beträgt maximal 7 min, vorzugsweise maximal 3 min, noch bevorzugter maximal 1 min, insbesondere maximal 0.5 min, am bevorzugtesten maximal 0.2 min, minimal beträgt die Zeitdauer 5sec. Kurze Überführungszeiten sind insbesondere bei hohen Temperaturen T_{LÜ} zur Unterdrückung von thermischem Abbau von Bedeutung.
5. Die Aufheizgeschwindigkeit v_{d} beim Überführen in Schritt d) beträgt mindestens 1°C/min, vorzugsweise mindestens 10°C/min, noch bevorzugter mindestens 50°C/min, insbesondere mindestens 100°C/min, am bevorzugtesten mindestens 200°C/min, maximal beträgt die Aufheizgeschwindigkeit etwa 300°C/min. Hohe Aufheizgeschwindigkeiten sind insbesondere bei hohen Konzentrationen von netzwerkfähigen Stärken, bei hohen Molekulargewichten dieser Stärken, bei hoher Temperatur T_{LÜ} in Schritt d) und zur Unterdrückung eines thermischen Abbaus von netzwerkfähigen Stärken wichtig.
6. Die Temperatur T_{L1} Schritt e) in beträgt maximal 0.9 T_{LÜ}, vorzugsweise maximal 130°C, noch bevorzugter maximal 100°C, insbesondere maximal 70°C, am bevorzugtesten maximal 30°C. Die minimale Temperatur liegt bei etwa 0°C. Tiefe Temperaturen T_{L1} sind zur Einstellung hoher Unterkühlungen und zur Einstellung hoher Keimzahlen von Bedeutung. Dies ist allgemein für die Herstellung hochfester Netzwerke mit tiefsten Quellgraden wichtig, da für Stärke-Gel Weichkapseln hohe Quellgrade eingestellt werden sollen, ist die Unterkühlung von netzwerkfähige Stärke enthaltenden Lösungen nur von untergeordneter Bedeutung.
7. Die Zeitdauer delta tₑ des Überführens in Schritt e) beträgt maximal 7 min, vorzugsweise maximal 3 min, noch bevorzugter maximal 1 min, insbesondere maximal 0.5min, am bevorzugtesten maximal 0.2min, die kürzesten Zeiten liegen bei etwa 5 sec. Kurze Zeiten sind im Zusammenhang mit hochfesten Netzwerken wichtig, um hohe Unterkühlungen delta T_{LU} und damit hohe Keimzahlen Z_{K} zu erhalten, ohne dass dabei eine Netzwerkbildung oder Kristallisation der netzwerkfähigen Stärke einsetzt. Für Stärke-Gel Weichkapseln sind diese Parameter und Effekte sekundär.
8. Die Abkühlgeschwindigkeit vₑ beim Überführen in Schritt e) beträgt mindestens 5°C/min, vorzugsweise mindestens 30°C/min, noch bevorzugter mindestens 70°C/min, insbesondere mindestens 110°C/min, am bevorzugtesten mindestens 200°C/min, maximal beträgt die Abkühlungsgeschwindigkeit etwa 300°C/min. Durch hohe Abkühlungsgeschwindigkeiten kann eine hohe Keimzahl im zweiten Fluid Z_{K} erreicht werden, ohne dass dabei eine Netzwerkbildung oder Kristallisation der netzwerkfähigen Stärken einsetzt.
9. Der pH in den Schritten d) und e) liegt für Stärke im Bereich 5 - 12, vorzugsweise im Bereich 6 -12, noch bevorzugter im Bereich 7 bis 12. Ein erhöhter pH erleichtert die Löslichkeit von netzwerkfähiger Stärke. Gegebenenfalls wird der pH der Gesamtmischung in Schritt g) durch Zugabe einer Säure oder Base auf den gewünschten Wert, vorzugsweise auf pH 6 - 8 eingestellt.
10. Die Schergeschwindigkeit G_{d} in den Schritten d) und/oder e) und f) beträgt mindestens 10/s, vorzugsweise mindestens 100/s, noch bevorzugter mindestens 1000/s, insbesondere mindestens 10'000/s, am bevorzugtesten mindestens 50'000/s. Maximale Schergeschwindigkeiten liegen bei rund 100'000/s. Durch hohe Schergeschwindigkeiten kann in Schritt d) die Löslichkeit insbesondere von netzwerkfähigen Stärken mit hohem Molekulargewicht deutlich verbessert werden und können somit höhere Konzentrationen verarbeitet werden. In Schritt e) verhindern hohe Schwergeschwindigkeiten eine vorzeitige Netzwerkbildung.

Entsprechend den obigen Bedingungen 1 bis 10 behandelte netzwerkfähige Stärken werden anschliessend mit vorliegenden Stärken gemischt, um Netzwerke zu erhalten, wobei sowohl netzwerkfähige Stärken als auch vorliegende Stärken einen Beitrag zum entstehenden Netzwerk leisten.

Nachdem eine netzwerkfähige Stärke oder eine Mischung von netzwerkfähigen Stärken entsprechend obigen Bedingungen gelöst und gegebenenfalls unterkühlt worden sind, können sie direkt mit vorliegenden Stärken gemischt werden oder aber zwei oder mehrere Lösungen werden zuerst zusammengeführt, gemischt und dann vorliegenden Stärken zugeführt. Es ist in bestimmten Fällen auch möglich, aufbereitete netzwerkfähige Stärken in jeweils verschiedene erste Fluide von vorliegenden Stärken einzumischen und diese Mischungen dann zu einer Gesamtmischung zu vereinigen.

### 2. Mischen von vorliegender Stärke mit netzwerkfähiger Stärke

Insbesondere um einphasige Gele zu erhalten, ist eine molekulardisperse Mischung von vorliegender Stärke und netzwerkfähiger Stärke eine wesentliche Voraussetzung. Solche Mischungen können durch Anwendung von Scherung und hohen Schergeschwindigkeiten erhalten werden. Ist eine molekulardisperse oder nahezu molekulardisperse Mischung eingestellt worden, kann eine Phasenseparation durch die kinetische Kontrolle des Prozesses eingeschränkt oder ganz verhindert werden. Dies bedeutet eine entsprechende Kontrolle der Abkühlungsgeschwindigkeit, wobei der einphasige thermodynamisch metastabile Zustand eingefroren werden kann.
1. Der Weichmachergehalt WM₁ in Gew.% der vorliegenden Stärke in Schritt c) vor dem Zuführen der netzwerkfähigen Stärke beträgt 5% - 90%, vorzugsweise 5% - 70%, noch bevorzugter 5% - 60%, insbesondere 5% - 50%, am bevorzugtesten 5% - 45%.
2. Der durchschnittliche Verzweigungsgrad Q_{b} der Stärke-Mischung in Schritt g) liegt infolge der Mischung mit meist deutlich stärker verzweigten vorliegenden Stärken üblicherweise höher als der durchschnittliche Verzweigungsgrad der eingesetzten netzwerkfähigen Stärke, Q_{b} ist kleiner als 0.05, vorzugsweise kleiner 0.02, noch bevorzugter kleiner als 0.006, insbesondere kleiner als 0.003, am bevorzugtesten kleiner als 0.001.
3. Der Weichmachergehalt WM₂ in Gew.% unmittelbar nach Schritt g) ist kleiner als 80%, vorzugsweise kleiner als 75%, noch bevorzugter kleiner als 70%, insbesondere kleiner als 65%, am bevorzugtesten kleiner als 60%. Der minimale Weichmachergehalt WM₂ liegt bei 15%.
4. Die Schergeschwindigkeit G_{g} beim Mischen von erstem Fluid mit zweitem Fluid beträgt mindestens 10/s, vorzugsweise mindestens 100/s, noch bevorzugter mindestens 1000/s, insbesondere mindestens 10'000/s, am bevorzugtesten mindestens 50'000/s. Die maximale Schergeschwindigkeit liegt bei etwa 100'000/s. Durch hohe Schergeschwindigkeiten wird bevorzugt eine mokelulardisperse Mischung der Fluide erreicht, was für hohe resultierende Netzwerkdichten N₀/V₀ und insbesondere für einphasige Netzwerke eine Voraussetzung ist. Ausserdem wird durch hohe Schergeschwindigkeiten G_{g} eine grosse Zahl von möglichst kleinen die Netzwerkelemente bildenden Kristalliten erhalten.
5. Zusätzlich kann die Netzwerkdichte nach erfolgter Netzwerkbildung in der Mischung durch geeignete Fremdnukleierungsmittel erhöht werden. Die Anzahl der bei der Netzwerkbildung wirksamen Keime Z ist dann gegeben durch Z = Z_{K} + Z_{N}, wobei Z_{K} die Zahl der Keime im zweiten Fluid und Z_{N} die Zahl der Fremdkeime ist.
6. Die Konzentration der entsprechend den Schritten d) bis f) verarbeiteten netzwerkfähigen Stärke in der Mischung von Schritt g) in Gew.% beträgt 1% - 95%, vorzugsweise 2% - 70%, noch bevorzugter 3% bis 50%, insbesondere 3% bis 30%, am bevorzugtesten 3% - 25%. Durch Anwendung hoher Konzentrationen von netzwerkfähigen Stärken im zweiten und dritten Fluid können nach erfolgter Mischung mit vorliegenden Stärken entsprechend hohe Konzentrationen von netzwerkfähigen Stärken in der Mischung erhalten werden, wodurch sich hohe Netzwerkdichten und damit hohe Festigkeiten des Netzwerks einstellen lassen.

In mindestens einem der Schritte a) bis g) kann mindestens ein Weichmacher mindestens teilweise aus dem Prozess entfernt werden, dies ist insbesondere bei Schritt g) wichtig, da durch die Reduktion des Weichmachergehalts die Phasenseparation unterdrückt werden kann, indem die Mobilität der Moleküle eingeschränkt wird.

### 3. Filmbildung, Umformung und Netzwerkbildung

Nachdem die Dispergierung der netzwerkfähigen Stärken im ersten Fluid stattgefunden hat, die Zuschlagstoffe eingemischt sind und der Weichmachergehalt WM₃ eingestellt ist, die Mischung die Temperatur T₃ erreicht hat, wird daraus ein Film hergestellt. Der Film kann in Längsrichtung zweigeteilt werden und die zwei Hälften können danach einer Umformanlage zugeführt werden, wobei die zwei Hälften der dabei hergestellten, gefüllten und verschweissten Weichkapseln aus den zwei Film-Hälften stammen. Alternativ können auch parallel jeweils zwei Filme hergestellt werden, die dann der Umformanlage zugeführt werden. Die Netzwerkbildung wird frühestens kurz vor oder während der Umformung zu resultierenden Weichkapseln durch ein Absenken der Temperatur eingeleitet. Eine weitere Möglichkeit zur Steuerung des Beginns der Netzwerkbildung besteht in der Auswahl und in der Konzentration der netzwerkfähigen Stärken, wobei ein breiter Spielraum bezüglich der Temperatur, bei der die Netzwerkbildung oder Gelierung einsetzt, zur Verfügung steht. Eine weitere Möglichkeit zur Einflussnahme auf die Gelierungstemperatur besteht in der Wahl der Lösungs- bzw. Ueberhitzungstemperatur, der Unterkühlung und weiterer Parameter der Verfahrensschritte d) und e). Während der Umformung zu resultierenden Weichkapseln, wobei hohe Dehnungen zur Anwendung kommen, darf die Netzwerkbildung keinesfalls vollständig sein, weil dies unweigerlich zu einem Zerreissen des Materials führen würde. Ein geringer Anteil an Netzwerkbildung von einigen Prozent bezüglich voll entwickeltem Netzwerk jedoch kann vorteilhaft sein, weil dadurch die Strukturviskosität der Schmelze und damit deren Dehnbarkeit verbessert wird. Der Prozess wird so gesteuert, dass die Netzwerkbildung zur Hauptsache nach der Verschweissung der Weichkapselhälften erfolgt. Nach diesem Zeitpunkt ist eine möglichst schnelle Netzwerkbildung vorteilhaft. Dieser kann beispielsweise dadurch beschleunigt werden, dass die resultierenden Weichkapseln in einem Kaltluftstrom bei tiefer Luftfeuchtigkeit kurzzeitig gekühlt werden. Dadurch gewinnen die Weichkapseln schnell an Festigkeit und deren Oberfläche zeigt infolge der Gelierung praktisch keine Klebrigkeit, wodurch die weitere Behandlung der Kapseln vereinfacht wird.

Gele oder Netzwerke mit tiefen Weichmachergehalten sind transparent, weil die Grösse der Kristallite unterhalb der Wellenlänge des sichtbaren Lichts liegt und die Kristallite das Licht deshalb nicht zu streuen vermögen. Dies ist ein Indiz dafür, dass es durch die getroffenen Massnahmen gelungen ist, eine sehr kleine Kristallitgrösse einzustellen. Solche transparente Gele werden als einphasige Gele bezeichnet. Bei höheren Weichmachergehalten bilden sich grössere Kristallite aus, deren Grösse in der Grössenordung der Wellenlänge des sichtbaren Lichts oder grösser ist, die deshalb das Licht zu streuen vermögen, also nicht transparent sind und eine milchig weisse Farbtönung aufweisen, wie dies bei herkömmlichen Gelen beobachtet werden kann. Die Steuerung der Transparenz erfolgt jedoch nicht nur über den Weichmachergehalt, entscheidend ist auch der Dispergierungsgrad der Lösung netzwerkfähiger Stärke, deren Konzentration in der Gesamtmischung, die Viskosität und insbesondere die Parameter in den Schritten d) und e).

Die Schritte a) bis k) werden vorzugsweise zumindest in Teilbereichen kontinuierlich durchgeführt, wobei die geeignete Verfahrenszone der Prozessraum mindestens eines Mischers ist und die Schritte a) bis g) kontinuierlich in aufeinander folgenden Abschnitten des mindestens einen Mischers und die Schritte h) und i) in einer dem mindestens einen Mischer nachgeschalteten Formgebungs- bzw. Umformungseinheit stattfinden. Der mindestens eine Mischer kann ein Einschnecken- oder ein Zweischnecken- oder ein Mehrschnecken- oder ein Ringextruder oder ein Kokneter oder ein statischer Mischer oder ein Ystral Mischer oder eine Rührwerkskugelmühle oder eine andere bezüglich Temperatur, Druck und Scherung regelbare Verfahrensstrecke sein. Für die Herstellung des Films können die gängigen thermoplastischen Formgebungsverfahren herangezogen werden können, beispielsweise die Extrusion durch eine Breitschlitzdüse und nachfolgend Formwalzen. Die Umformanlage ist vorzugsweise ein kontinuierlich arbeitende Verkapselungsanlage, beispielsweise eine Rotary-Die Anlage. In einer weiteren Verfahrensvariante werden vorgängig die Schritte a) bis c) durchgeführt, wobei ein Granulat von Thermoplastischer Stärke erhalten wird, das transportiert und zwischengelagert werden kann. Die Thermoplastische Stärke wird danach erneut in eine Schmelze überführt, worauf diese Schmelze mit einer oder mehreren Lösungen von netzwerkfähigen Stärken, die entsprechend den Schritten d) und e) vorbereitet werden, in den Schritten f) und g) gemischt werden können.

Bezüglich der Form der Weichkapseln sind keine Grenzen gesetzt, es kann sich dabei um eine beliebige Form handeln, ausserdem sind auch Zwei- und Mehrkammerkapseln herstellbar. Als Füllgut können Füllgüter entsprechend dem Stand der Technik eingesetzt werden wie flüssig- bis pastöse Stoffe, Pulver, Kügelchen, Granulat etc.. Neben Weichkapseln können auch Paint-Balls und weitere Produkte erzeugt, die entsprechend dem Stand der Technik mittels Weichgelatine-Verkapselungstechniken hergestellt werden. Ausserdem kann der mit dem vorgeschlagenen Verfahren hergestellte Film eine Schicht von Mehrschicht-Formkörpern, insbesondere von Mehrschicht-Weichkapsel sein. Weitere Schichten können beispielsweise aus PEG, Alginaten, Carrageenanen oder modifizierter Cellulose wie HPMC bestehen.

### Weichmacher

Als Weichmacher können grundsätzlich dieselben Lösungsmittel, Weichmacher und Weichmachermischungen verwendet werden, die entsprechend dem Stand der Technik als Lösungsmittel, Weichmacher und Weichmachermischungen für Stärke oder Thermoplastische Stärke geeignet sind, bevorzugt werden sie aus folgender Gruppe ausgewählt:
➢ Wasser; Glycerin; Glycerinethoxylat; Polyglycerine; Di-, bis Decaglycerine; Polyglycerinmonoethoxylate; Reaktionsprodukte von Glucose mit Ethylenoxid; Glucosemonoethoxylat; Glucoside; Butylglucosid; Aphamethylglucosid; Maltose, Glucotri- und höhere Glucopolysaccharide, Mono- und Oligosaccharid-Sirupe; Alkohole; Polyalkohole; Butanol; Erythritol; Pentaerythritol; Triethylolpropan; Trimethylolpropan; Triethylpropanmonoethoxylat; Propandiole; Butandiole; Pentandiole; Hexandiole; Hexantriole; Polyvinylalkohole mit 3 bis 20 Monomereinheiten; ganz oder teilweise zu Polyvinylalkoholen hydrolysierte Polyvinylacetate; Trihydroxymethylaminomethane; Aminoalkohole; Fettalkohole; Amine; Hydroxyalkydamin; Ethylendiamin; Amide; Esteramide; Formamid; Säureamide; Sulfoxide; DMSO; quaternäre Ammonium-Verbindungen; Glycol, Ethylenglycol; Ethylendiglycol; Ethylentriglycol; Propylenglycol; Propylendiglycol; Propylentriglycol; Neopentylglycol; Polyethylenglykole; Polypropylenglycol; Polyglycole; Pyrrolidone; 2-Pyrrolidon oder 1-Methyl-2-Pyrrolidon; Caprolactan; Polycaprolactan; Sorbitol; Sorbitolacetat; Sorbitoldiacetat; Sorbitolmonoethoxylat; Sorbitoldipropoxylat; Sorbitoldiethoxylat; Sorbitolhexaethoxylat; Salze von Carboxymethylsorbitol; Aminosorbitol; Maltitol; Mannitol; Mannitolmonoacetat; Mannitolmonoethoxylat; Xylitol; Arabitol; Adonitol; Iditol; Galactitol; Allitol; Säuren; Carboxylsäuren; Ameisensäure; Essigsäure; Bernsteinsäure; Bernsteinsäureanhydrid; Adipinsäure; Milchsäure; Weinsäure; Citronensäure: Apfelsäure; Hydroxybuttersäure; Maleinsäure; Fettsäuren; Dimethylsulfoxid; Harnstoff; chemisch veränderte, insbesondere durch Veresterung erhaltenen Elemente dieser Gruppe; Mischungen von Elementen dieser Gruppe.

Weichmacher oder Weichmachermischungen werden üblicherweise den vorliegenden Stärken in Schritt b) und den netzwerkfähigen Stärken in Schritt d) zugeführt, zusätzlicher Weichmacher kann ausserdem auch in mindestens einem der Schritte a), c), e) f) oder g) dem Verfahren zugeführt werde. Die Zuführung von Weichmacher in Schritt b) kann entfallen, wobei dann der Schritt c) ebenfalls entfällt und die entsprechende vorliegende Stärke in Schritt g) gleichzeitig mit dem Mischen zu der Gesamtmischung in ein Fluid überführt, bzw. plastifiziert wird.

Weichmacher kann gegebenenfalls in mindestens einem der Schritte aus dem Verfahren entfernt werden, beispielsweise durch Entgasungstechniken, insbesondere in mindestens einem der Schritte f) und g). Dies ist insbesondere für die Herstellung von Weichkapseln mit niederem Weichmachergehalt und hoher Quellfestigkeit von Bedeutung (Controlled Release Kapseln).

Im Vergleich zu WO 01/37817 A1 können infolge der teilkristallinen, bzw. mikrokristallinen Gel Struktur, weitere Weichmacher wie beispielsweise Sorbitol, Maltitol oder Mannitol in geringeren Anteilen eingesetzt werden, sodass das Sorptionsverhalten und damit die Produkteigenschaften unter wechselnden Bedingungen verbessert werden können.

### Zusätze

### 1. Fremdnukleierungsmittel

Fremdnukleierungsmittel können insbesondere bei tiefen Weichmachergehalten WM₀ in mindestens einem der Schritte a) bis g) dem Prozess zugeführt werden, um die Netzwerkbildung unter erschwerten Bedingungen zu erleichtern und die Netzwerkdichte zu erhöhen. Sie werden aus folgender Gruppe ausgewählt:
> Nanopartikel; Nanopartikel von Mono-, Oligo- und Polysacchariden; mikrokristalline Cellulose; oberflächenbehandelte mikrokristalline Zellulose; Polysaccharid-Mikrokristallite; Stärke-Mikrokristallite; mineralische Mikro- und Nanokristallite wie beispielsweise Bornitrid, Sorbitol-Derivate, insbesondere 3,4-dimethyldibenzyliden-Sorbitol; Titanoxid; Calziumcarbonat; Nanoclays; Mischungen von Elementen dieser Gruppe.

### 2. Keimstabilisatoren

Keimstabilisatoren können der Mischung der netzwerkfähigen Polysaccharide in mindestens einem der Schritte d) bis f) zugeführt werden, um insbesondere bei hochkonzentrierten Fluiden netzwerkfähiger Stärke das Kristallitwachstum zu unterdrücken. Im Allgemeinen werden als Keimstabilisatoren stark verzweigte Polysaccharide verwendet, welche keine Gelbildung zeigen oder erst nach Tagen oder Wochen nur sehr schwache Gele bilden. Beispiele sind Glycogen, Amylopektin oder Agaropektin. Vorzugsweise werden Amylopektine mit einem Blue-Value von kleiner als 0.08 und/oder mit einer lod-Affinität von kleiner als 0.7g/100g eingesetzt.

### 3. Additive

Additive können in mindestens einem der Schritte a) bis g) zur Verbesserung der Verarbeitbarkeit, zur Beeinflussung der Netzwerkbildung und zur Modifikation der Produkteigenschaften mit Anteilen in Gew.% von 0.01% bis 10%, vorzugsweise von 0.02% bis 7%, noch bevorzugter von 0.03% bis 5% zugeführt werden. Unter anderem können Additive und Hilfsstoffe, welche bei der Herstellung von Thermoplastischer Stärke dem Stand der Technik entsprechen, auch für Stärke-Gele eingesetzt werden. Additive werden insbesondere aus folgender Gruppe von Stoffen ausgewählt:
➢ Lebensmitteladditive, insbesondere Antioxidantien und Lebensmittel-Stabilisatoren; Glycerinderivate; Mono-, Di- und Triglyceride und deren Stearate; Glycerinmonostearat; Mono-, Di-, oder Triglyceride von Speisefettsäuren; Polyglycerinester, insbesondere der Speisefettsäuren; Polyethylen-glycole; Polyethylenglycolester, insbesondere der Speisefettsäuren; Lecithine; nichtionische und ionische Netzmittel und Tenside; Emulgatoren; Komplexbildner; Amylose-Komplexbildner; Na-2-stearoyl-Lactat; aliphatische Alkohole; Fettsäuren, insbesondere Stearinsäuren, aliphatische und aromatische Ester; Pyridin; Zucker; Zuckerester, insbesondere Zuckerester der Sepeisefettsäuren; Fette; Fettsäurenester; Wachse, insbesondere vegetarische Wachse wie Carnauba-Wachs, Candelillawachse, Japanwachs, Ouricurywachs, Gagelstrauchwachs, Jojobawachs; Polyolefinwachse; Naturharze; Schellack; Chitin; Kollagen; Casein; Mono- und Oligosaccharide; Dextrane; Proteine; Peptide; Polypeptide, insbesondere pflanzliche Polypeptide; Cellulose, Cellulosederivate, insbesondere hydroxypropylierte Cellulose; Hydrocolloide, insbesondere Alginate, Carrageenane, Galactomannane, Glucomannane; Farbstoffe; als Lebensmittel verwendbare Stoffe; Aromastoffe; Mischungen von Elementen dieser Gruppe.

### 4. Füllstoffe

Füllstoffe können in mindestens einem der Schritte a) bis g), zugeführt werden, um Eigenschaften des Werkstoffs zu modifizieren und/oder die spezifischen Rohstoffkosten per Kilo zu reduzieren. Allgemein kommen Füllstoffe in Frage, welche entsprechend dem Stand der Technik in der Kunststoff- und Biokunststofftechnik eingesetzt werden, insbesondere werden sie werden aus folgender Gruppe ausgewählt:
➢ Mineralien, insbesondere Titandioxid, Talkum, Clays; Holzmehl; Lignin; Fasern, insbesondere Naturfasern wie Baumwolle, Hanffasern, Flachs, Ramie, Jutefasern; Russ; Clays; native Stärke; inhibierte Stärke; vernetzte Stärke; Stärke mit einem Amylose-Gehalt von mehr als 40%; Mischungen von Elementen dieser Gruppe.

### 5. Sprengmittel

Als Sprengmittel können entsprechend dem Stand der Technik in der Galenik verwendete Stoffe verwendet werden, wie beispielsweise Carbonate und Hydrogencarbonate der Alkali- und Erdalkaliionen, insbesondere Calciumcarbonat. Ausserdem kommen auch Amylasen in Frage. Ein Sprengmittel oder Mischungen von Sprengmitteln können in einem der Schritte a) bis c) oder g) zudosiert werden.

### 6. Spezielle Zusätze

Durch spezielle Zusätze von gummiartigen Stoffen, insbesondere von Hydrokolloiden, kann die Zähigkeit der Gele massiv verbessert werden, indem der als eine separate Phase in der Stärke-Gel-Matrix vorliegende spezielle Zusatz Spannungsspitzen auffangen kann. Die speziellen Zusätze werden vorzugsweise aus der folgenden Gruppe ausgewählt:
➢ Galactomannane, wie Guar-Gummi oder Johannisbrotkernmehl; Pectine, insbesondere Rhamnogalakturonane und Protopektine; Dextrane; Xanthan; Zymosan; Hydrokolloide aus Meeresalgen, wie Alginate, Agar-Agar, Agarose, Carrageen und Carrageenane; Furcellaran; Hydrokolloide aus Flechten, wie Lichenine und Isolichenine, oder Hydrokolloide als Exsudate aus Hölzern, wie Tragant (Astragalus Gummi), Karaya-Gummi, Gummi-Arabicum, Kutira-Gummi; Inulin; Latex; Chitin; Chitosan; Kollagen; Casein; Mischungen von Elementen dieser Gruppe.

Um optimale Ergebnisse zu erhalten, ist eine möglichst feine Verteilung dieser Phase in der Matrix entscheidend. Bei gleichem Anteil an speziellem Zusatz ist der Gewinn an Zähigkeit entscheidend von dessen Verteilung in der Matrix und der Partikelgrösse abhängig. Dies wird einerseits ermöglicht, indem der spezielle Zusatz als möglichst feines Pulver vorbereitet wird, andererseits indem dieser Zusatz vorgängig gequollen wird und dann zu der vorliegenden Stärke im nativen Zustand bei geringem Weichmachergehalt zugegeben wird. Durch die bei der Mischung wirkenden Scherkräfte werden die gequollenen weichen Partikel des speziellen Zusatzes durch die harten nativen Stärkekörner fragmentiert und gemahlen, sodass sich eine entsprechend fein verteilte Phase der speziellen Zusätze erhalten lässt.

Die Bedingungen der Zumischung der speziellen Zusätze zur Einstellung einer hochdispersen Phase der speziellen Zusätze sind:
A. Die speziellen Zusätze weisen einen Weichmachergehalt in Gew.% im Zeitpunkt der Zuführung von 5% - 90%, vorzugsweise von 11% - 90%, noch bevorzugter von 18% - 90%, insbesondere von 26% - 90%, am bevorzugtesten von 33% - 90% auf. Bevorzugt wird Wasser als Weichmacher bzw. Quellmittel verwendet
B. Der Mittelwert der Partikelgrössenverteilung der speziellen Zusätze bei 5% bis 20% Wassergehalt liegt im Bereich 150mü - 0.1mü, vorzugsweise im Bereich 100mü - 0.1mü, noch bevorzugter im Bereich 50mü - 0.1mü, insbesondere im Bereich 10mü - 0.1 mü, am bevorzugtesten im Bereich 5mü - 0.1 mü (1 mü = 1 mikrometer = 1□m).
C. Die speziellen Zusätze werden zu vorliegenden Stärken im nativen, pregelatiniserten, teilweise oder ganz plastifizierten Zustand in mindestens einem der Schritte a) bis c) zugegeben, vorzugsweise im Schritt a), während der Weichmachergehalt der vorliegenden Stärken in Gew.% zu diesem Zeitpunkt im Bereich 1% - 50%, vorzugsweise im Bereich 1% - 30%, noch bevorzugter im Bereich 1% - 20%, insbesondere im Bereich 1% - 15%, am bevorzugtesten im Bereich 1% - 12% liegt.

Durch optimale Verfahrensweise kann ein spezieller Zusatz als hochdisperse Phase in der Matrix dispergiert werden, wobei die mittlere Grösse dieser Phase im Bereich 50mü - 0.07mü, vorzugsweise im Bereich 20mü - 0.07mü, noch bevorzugter im Bereich 7mü - 0.07mü, insbesondere im Bereich 3mü - 0.07mü, am bevorzugtesten im Bereich 1 mü - 0.07mü liegt.

Das Zumischen der speziellen Zusätze unter den angegebenen Bedingungen ist neben der Herstellung von zähen Stärke-Gel Weichkapseln auch für die Herstellung von TPS-Weichkapseln mit verbesserter Zähigkeit vorteilhaft.

### 7. Resistente Stärken

Um die prebiotische Wirkung von auf Stärke-Gel basierenden Weichkapseln zu erhöhen und das Sorptionsverhalten zu verbessern, kann zusätzliche resistente Stärke, vorzugsweise in Form eines feinen Pulvers als Zuschlagstoff in mindestens einem der Schritte a) bis g) zugemischt werden. Sie kann insbesondere aus der folgenden Gruppe ausgewählt werden:
Resistente Stärken 1. Art, resistente Stärken 2. Art, resistente Stärken 3. Art, auf Stärke-Gel basierende resistente Stärke, Kombinationen von Elementen dieser Gruppe.

### Strukturtyp von Stärke-Netzwerken

Durch geeignete Prozessführung kann erreicht werden, dass die sich bildenden Kristallite bei Raumtemperatur bevorzugt eine A-Struktur aufweisen. Dieser Strukturtyp zeigt gegenüber dem bei Raumtemperatur stabilen B-Strukturtyp bei gleicher Luftfeuchtigkeit eine massiv reduzierte Wasseraufnahme, wodurch sich ein günstigeres Sorptionsverhalten ergibt. Der bei Raumtemperatur metastabile A-Strukturtyp kann durch kinetische Kontrolle eingefroren und so auch bei Raumtemperatur erhalten werden. Eine weitere Möglichkeit zur Einstellung des A-Strukturtyps ist gegeben durch eine Wärmebehandlung, wobei der B-Strukturtyp in den gewünschten A-Strukturtyp umgewandelt wird. Die notwendige Temperatur, die nur kurzzeitig angewendet werden muss, liegt oberhalb von 100°C.

### Eigenschaften der Weichkapseln

Der Quellgrad Q (Q = Volumen nach der Quellung/Volumen vor der Quellung) der bei 50% Luftfeuchtigkeit und 25°C konditionierte Weichkapselhülle beim Einlegen in Wasser bei 25°C im maximal gequollenen Zustand liegt im Bereich 1.1 - 20, vorzugsweise im Bereich 1.1 - 10, am bevorzugtesten im Bereich 1.1 - 7. Für Controlled-Release Kapseln liegt der Quellgrad Q im Bereich 1.03 bis 7, vorzugsweise im Bereich 1.03 bis 5, am bevorzugtesten im Bereich 1.03 bis 3.

Die Bruchfestigkeit der bei 50% Luftfeuchtigkeit und 25°C konditionierten Weichkapselhüllen liegt im Bereich 1MPa - 30MPa, vorzugsweise im Bereich 1.2MPa - 20MPa, am bevorzugtesten im Bereich 2MPa bis 17MPa.

Die Bruchdehnung der bei 50% Luftfeuchtigkeit und 25°C konditionierten Weichkapselhülle liegt im Bereich 10% - 200%, vorzugsweise im Bereich 15% bis 150%, am bevorzugtesten im Bereich 20% - 125%

Der Gesamtweichmachergehalt der Weichkapselhüllen in Gew.% nach Konditionierung bei 50% Luftfeuchtigkeit und 25°C liegt im Bereich 10% - 70%, vorzugsweise im Bereich 14% - 60%, am bevorzugtesten im Bereich 18% - 50%.

Im Vergleich zu Weichkapselhüllen basierend auf Thermoplastischer Stärke zeigen Weichkapselhüllen entsprechend dem vorgeschlagenen Verfahren einen flacheren Verlauf der Sorptionskurve (Wassergehalt in Funktion der Wasseraktivität). Bei gleicher Wasseraktivität werden tiefere Wassergehalte erhalten. Besonders ausgeprägt ist dieses Verhalten bei Wasseraktivitäten oberhalb 0.5, insbesondere oberhalb 0.7.

### Parameter

| | |
|---|---|
| T_{L0} | Minimale Temperatur, bei der sich die netzwerkfähigen Stärken lösen |
| T_{LR} | Temperatur der Rekristallisation der netzwerkfähigen Stärken im thermodynamischen Gleichgewicht nach dem Lösen bei T_{L0} |
| T_{LÜ} | Überhitzungstemperatur T_{LÜ} > T_{L0} |
| T_{LM} | Temperatur bei der der metastabile Zustand des unterdrückten Keimwachstums für 10 Sekunden aufrechterhalten werden kann |
| delta T_{LU} | Unterkühlung delta T_{LU} = T_{LR} - T_{LM} |
| T_{L1} | Temperatur der Lösung beim Einmischen des zweiten oder dritten Fluids in das erste Fluid, T_{LM} < T_{L1} < T_{LÜ}, insbesondere T_{LM} < =T_{L1} < T_{LR} |
| T₁ | Temperatur des ersten Fluids vor der Zuführung des zweiten oder dritten Fluids |
| T_{M} | Temperatur während des Mischvorgangs |
| T₃ | Temperatur am Ende des Mischvorgangs |
| T_{K} | Temperatur zu Beginn der Netzwerkbildung |
| delta t_{d} | Zeitdauer des Ueberführens in Schritt d) |
| delta tₑ | Zeitdauer des Ueberführens in Schritt e) |
| v_{d} | Aufheizgeschwindigkeit in Schritt d) |
| vₑ | Aufheizgeschwindigkeit in Schritt e) |
| Z_{K} | Anzahl der Keime im dritten Fluid bei T_{L1} |
| Z_{N} | Zahl der Fremdkeime in der Mischung von erstem und zweitem oder dritten Fluid |
| Z | Zahl der bei der Netzwerkbildung aktiven Keime |
| C_{PN} | Konzentration der netzwerkfähigen Stärke im zweiten oder dritten Fluid |
| C_{PNM} | Konzentration der netzwerkfähigen Stärke in der Mischung |
| C_{Sta} | Konzentration des Keimstabilisators im ersten Fluid |
| C_{StaM} | Konzentration des Keimstabilisators in der Mischung |
| C_{N} | Konzentration des Fremdnukleierungsmittels in der Mischung |
| WM_{d} | Weichmachergehalt in Schritt d) |
| WM₁ | Weichmachergehalt der vorliegenden Stärken zu Beginn des thermoplastischen Verfahrens |
| WM₂ | Weichmachergehalt der Mischung nach Zugabe des zweiten oder dritten |
| | Fluids |
| WM₃ | Weichmachergehalt am Ende des Mischvorgangs |
| WM₀ | Weichmachergehalt bei der Netzwerkbildung |
| W₀ | Wassergehalt bei der Netzwerkbildung |
| W₁ | Wassergehalt nach Quellung eines Films mit W₀ in Wasser |
| G_{d} | Schergeschwindigkeit in Schritt d) |
| G_{g} | Schergeschwindigkeit in Schritt g) |
| p_{w}(T) | Wasserdampfdruck bei der Temperatur T |
| N₀/V₀ | Netzwerkdichte nach erfolgter Netzwerkbildung |
| DP | Mittlerer Polymerisationsgrad |
| CL | Mittlere Kettenlänge (Anzahl Monomereinheiten unverzweigter Kettensegmente) |
| Q_{b} | Durchschnittlicher Verzweigungsgrad: Anzahl Mole der verzweigten alpha-Glucan-Einheiten/Anzahl Mole der gesamten alpha-Glucan-Einheiten) |
| BV | Blue-Value |
| IA | lod-Affinität [g/100g] |
| M_{w} | Gewichtsmittel der Molekulargewichtsverteilung |
| T_{g} | Glasumwandlungstemperatur |

Die Weichmacher- und Wassergehalte beziehen sich jeweils auf vorliegende und netzwerkfähige Stärken, d.h. auf Stärken, welche konstituierende Bestandteile des Netzwerks sind. Ein Netzwerk enthaltend beispielsweise 10g vorliegende Stärke, 3g netzwerkfähige Stärke, 11g Wasser, 2g Glycerin, 7g Zucker und 5g eines Zusatzes weist daher einen Weichmachergehalt WM₀ von 100*(11+2)/(11+2+10+3) = 50% und einen Wassergehalt von 100*11/(11+10+3) = 45.8% auf.

### Beispiele

Weitere Vorteile, Merkmale und Anwendungsmöglichkeiten der Erfindung ergeben sich aus der folgenden Beschreibung von nicht einschränkend aufzufassenden Ausführungsbeispielen.

### Beispiel 1

### Rezeptur

67% (bezogen auf das Trockengewicht) vorliegende Stärke: 70% Kartoffelstärke (10% Wasser), nativ, 30% Maisstärke (10% Wasser), nativ
19% (bezogen auf das Trockengewicht) netzwerkfähige Stärke: 50% hochamylosehaltige Stärke, nativ (50% Amylosegehalt, 15% Wasser), 40% enzymatisch entzweigte Tapioka Stärke (15% Wasser), 10% Maltodextrin (15% Wasser)

Zusatz: 0.7% Lecithin, 0.5% Carnauba Wachs, 5% Calciumcarbonat, Zugabe zusammen mit vorliegenden Stärken

Spezielle Zusätze: 4.8% (bezogen auf das Trockengewicht) Xanthan (40% Wasser), 3% (bezogen auf das Trockengewicht) Latex (Emulsion), Zugabe vor Schritt b)

Weichmacher in Schritt b) 30% Wasser, 5% Sorbitol, 5% Maltitol (bezogen auf die vorliegenden Stärken, Trockengewicht)
Weichmacher in Schritt d) 75% Wasser, 5% Glycerin (bezogen auf die netzwerkfähigen Stärken, Trockengewicht)

### Verfahren

Doppelschneckenextruder, Zudosierung der netzwerkfähigen Stärken über Sulzermischer und thermisch geregelte Verfahrensstrecken mit Scherströmung, Lösung der netzwerkfähigen Stärken zusammen bei 190°C (1min, 30bar), pH 9, Unterkühlung bis 60°C (30sec). Einspritzen der unterkühlten Lösung in den Doppelschneckenextruder bei Massetemperatur der vorliegenden plastifizierten Stärke von 130°C, Mischung über rückfördernde Knetelemente, anschliessend Evakuierung, Schmelzpumpe und Breitschlitzextrusion, gefolgt von Chill-Roll Strecke, longitudinale Zweiteilung der Filme und Zuführen in Rotary-Die Anlage, Herstellung und Füllung der Formkörper, Konditionierung.

### Beispiel 2

Modifikation 1 von Beispiel 1: Separate Lösung/Unterkühlung/Einspritzung der hochamylosehaltigen Stärke (HAS) und der entzweigten Tapioka Stärke (TAS),
Weichmacher in Schritt d1) für HAS: 75% Wasser
Weichmacher in Schritt d2) für TAS: 70% Wasser, 10% Glycerin
HAS: Lösen bei 190°C (1min, 30bar, pH 9), Unterkühlen bis 80°C (20sec)
TAS: Lösen bei 200°C (1min, 20bar, pH 7), Unterkühlen bis 50°C (20sec)

### Beispiel 3

Modifikation 2 von Beispiel 1: Rezeptur identisch, jedoch Plastifizierung der vorliegenden Stärken und Einarbeiten der Zusätze und der speziellen Zusätze vorgängig in einem Schritt a) mittels Doppelschneckenextruder, anschliessend Granulierung.
Erneute Plastifizierung des Granulats in einem Einschneckenextruder, Lösung/Unterkühlung/Einspritzung der netzwerkfähigen Stärken in den Einschneckenextruder wie in Modifikation 1 von Beispiel 1, Mischung mittels Madoc-Element, keine Schmelzpumpe, die weiteren Verfahrensschritte sind identisch mit Beispiel 1.

## Patentansprüche

1. Verfahren zum Herstellen von Formkörpern auf Basis von Stärke-Gel, **dadurch gekennzeichnet, dass**
a. das Stärke-Gel aus einer Gesamtmischung von mindestens einer vorliegenden Stärke und mindestens einer netzwerkfähigen Stärke durch Homo- und/oder Heterokristallisation gebildet wird, worin die netzwerkfähige Stärke einen Verzweigungsgrad Q_{b} kleiner 0.01 aufweist, und
b. diese beiden Komponenten getrennt und individuell vorbereitet werden, wobei die vorliegende Stärke plastifiziert wird und die netzwerkfähige Stärke gelöst wird; und
c. die Komponenten in diesem Zustand nach ihrem Zusammenführen zur Gesamtmischung molekulardispers gemischt werden, und
d. die Gel- oder Netzwerkbildung höchstens teilweise vor oder während der Umformung der Gesamtmischung zu den resultierenden Formkörpern eingeleitet wird und hauptsächlich oder gänzlich nach der Umformung erfolgt.

2. Verfahren nach Anspruch 1, **dadurch gekennzeichnet, dass** das Verfahren folgende Schritte in mindestens einer Verfahrenszone aufweist:
a) Zuführen jeweils einer vorliegenden Stärke;
b) Einwirken jeweils eines ersten Weichmachers auf die jeweils eine vorliegende Stärke,
c) Überführen der jeweils einen vorliegenden Stärke in jeweils ein erstes Fluid, wobei jeweils eine erste Mischung gebildet wird;
d) Überführen jeweils einer netzwerkfähigen Stärke in jeweils ein zweites Fluid durch Einwirken jeweils eines zweiten Weichmachers;
e) Überführen des jeweiligen zweiten Fluids in ein jeweiliges drittes Fluid;
f) Einführen des jeweiligen zweiten Fluids aus Schritt d) und/oder des jeweiligen dritten Fluids aus Schritt e) in eine der jeweiligen ersten Mischungen aus den Schritten a) bis c);
g) Vereinigen der jeweiligen Mischungen aus den Schritten a) bis f) zu mindestens einer molekulardispersen Gesamtmischung;
h) Formen mindestens eines Films aus dem in Schritt g) gebildeten mindestens einen Gesamtgemisch;
i) Zuführen des in Schritt h) gebildeten mindestens einen Films in eine Umformungs-Anlage und Herstellung von resultierenden Formkörpern aus dem mindestens einen Film, insbesondere Zuführen des in Schritt h) gebildeten mindestens einen Films in eine kontinuierliche Verkapselungs-Anlage, beispielsweise einer Rotary-Die Anlage, und Herstellung von verschweissten, einen Füllstoff bzw. Wirkstoff enthaltenden Weichkapseln;
j) Einleitung der Bildung eines Stärke-Netzwerks aus der in Schritt g) gebildeten mindestens einen Gesamtmischung, durch Homokristallisation zwischen jeweiligen Makromolekülen der jeweils mindestens einen netzwerkfähigen Stärke untereinander und/oder durch Heterokristallisation zwischen diesen jeweiligen Makromolekülen und jeweiligen Makromolekülen der jeweils mindestens einen vorliegenden Stärke, nachdem die Schritte a) bis h) oder a) bis i) vollzogen sind.
k) Einstellung des gewünschten Weichmacher- bzw. Wassergehalts der resultierenden Formkörper, der Weichkapseln, durch Konditionierung bei bereitgestelltem zeitlichem Temperatur- und Luftfeuchtigkeitsverlauf.

3. Verfahren nach einem der Ansprüche 1 oder 2, **dadurch gekennzeichnet, dass** in mindestens einem der Schritte d) bis g), insbesondere nach Schritt f) und vor Schritt h) ein Weichmacher mindestens teilweise aktiv durch Evakuierungstechniken aus dem Verfahren entfernt wird.

4. Verfahren nach einem der Ansprüche 1 bis 3, **dadurch gekennzeichnet, dass** in Schritt d) das netzwerkfähige Stärke enthaltende zweite Fluid überhitzt und in Schritt e) das dritte Fluid unterkühlt wird.

5. Verfahren nach einem der vorangehenden Ansprüche 1 bis 4, **dadurch gekennzeichnet, dass** in mindestens einem der Schritte a) bis g) mindestens einmal ein Fremdnukleierungsmittel zugeführt und/oder das zweite oder dritte Fluid während oder nach Schritt e) mit Ultraschall behandelt wird.

6. Verfahren nach einem der vorangehenden Ansprüche 1 bis 5, **dadurch gekennzeichnet, dass** dem Verfahren in mindestens einem der Schritte a) bis g) mindestens ein Zusatz zugeführt wird.

7. Verfahren nach einem der vorangehenden Ansprüche 1 bis 6, **dadurch gekennzeichnet, dass** in mindestens einem der Schritte a) bis g), vorzugsweise in einem der Schritte a) bis c) ein spezieller Zusatz zugegeben wird und das resultierende Stärke-Gel mit hoher Zähigkeit diesen speziellen Zusatz in Form einer hochdispersen Phase enthält, wobei die mittlere Grösse dieser Phase im Bereich 50mü - 0.07mü, vorzugsweise im Bereich 20mü - 0.07mü, noch bevorzugter im Bereich 7mü - 0.07mü, insbesondere im Bereich 3mü - 0.07mü, am bevorzugtesten im Bereich 1mü - 0.07mü liegt.

8. Verfahren nach einem der vorangehenden Ansprüche 1 bis 7, **dadurch gekennzeichnet, dass** ein in Schritt h) gebildeter Film in Längsrichtung in zwei Hälften geteilt wird, die separat über gleich lange Wege und mit derselben Geschwindigkeit einer Rotary-Die Anlage zugeführt werden, sodass die zwei Hälften der verschweissten Weichkapseln eine identische Vorgeschichte erfahren haben, d.h. gleichzeitig und parallel in Schritt h) zu einem Film geformt wurden.

9. Verfahren nach einem der vorangehenden Ansprüche 1 bis 8, **dadurch gekennzeichnet, dass** die Schritte a) bis h) parallel in zwei verschiedenen Verfahrenszonen durchgeführt werden und die zwei Hälften der verschweissten Weichkapseln im Rotary-Die Verfahren aus zwei Filmen hergestellt werden, die aus den zwei verschiedenen Verfahrenszonen stammen.

10. Formkörper in Form einer Weichkapsel, **dadurch gekennzeichnet, dass** die Weichkapsel durch ein Verfahren gemäss einem der Ansprüche 1 bis 9 hergestellt worden ist.

11. Verwendung der Weichkapsel nach Anspruch 10 zur Herstellung von Controlled Release Anwendungen.

12. Mehrschicht-Formkörper in Form von Mehrschicht-Weichkapsel, **dadurch gekennzeichnet, dass** mindestens eine Schicht der Weichkapsel aus Stärke-Gel besteht, die durch ein Verfahren gemäss einem der Ansprüche 1 bis 9 hergestellt worden ist.

13. Weichkapsel, hergestellt nach einem Verfahren gemäss der Ansprüche 1 bis 9, **dadurch gekennzeichnet dass**, die Weichkapselhülle in Form eines Films von max. 0.3 mm Dicke und nachdem dieser Film getrocknet worden ist und in diesem Zustand in eine Atmosphäre mit maximal 43% Luftfeuchtigkeit bei Raumtemperatur gebracht und dort so lange aufbewahrt wird, bis das Gewicht des Films sich nicht mehr änderte und der Film einen Wassergehalt von W₄₃ aufweist und, nachdem der Film darauf in eine Atmosphäre mit mindestens 90% Luftfeuchtigkeit bei Raumtemperatur gebracht wird, bis das Gewicht sich nicht mehr ändert und der Film einen Wassergehalt von W₉₀ angenommen hat, die Differenz der Wassergehalte W₉₀ - W₄₃ in Gew.% 3% - 25% beträgt.

14. Weichkapsel nach Anspruch 13, **dadurch gekennzeichnet, dass** der kristalline Anteil der Weichkapselhülle, erhalten durch Separation des kristallinen Anteils vom amorphen Anteil der Weitwinkel-Röntgendiffraktionskurve einer Probe mit 7 bis 14 Gew.% Wasser, zwischen den Streuwinkeln 3° < 2theta< 37°, 15% - 100 beträgt.

15. Weichkapsel nach einem der Ansprüche 13 oder 14, **dadurch gekennzeichnet, dass** die Weichkapselhülle aus einphasigem, transparentem Stärke-Gel besteht, insbesondere im Rotary-Die Verfahren hergestellt worden ist.

16. Weichkapsel nach einem der vorangehenden Ansprüche 13 bis 15, **dadurch gekennzeichnet, dass** die Weichkapselhülle eine resistente Stärke in der Form eines Zuschlagstoffes enthält, insbesondere mit einem Anteil in Gew.% von 1% - 70%.

17. Weichkapsel nach einem der vorangehenden Ansprüche, **dadurch gekennzeichnet, dass** die Weichkapselhülle prebiotisch wirkt und/oder gegenüber einer TPS-Weichkapsel bei vergleichbarem Weichmachergehalt einen um 10% - 95% aufweist.

## Claims

1. Method for the production of mouldings based on starch gel, **characterized in that**
a. the starch gel is formed from a total mixture of at least one available starch and at least one starch having network capabilities by homo- and/or heterocrystallization, wherein the starch having network capabilities has a degree of branching Q_{b} of less than 0.01, and
b. these two components are prepared separately and individually, the available starch being plasticized and the starch having network capabilities being dissolved; and
c. the components in this state after their combination to give the total mixture being mixed to give a molecular disperse solution and
d. the gel or network formation is initiated at most partly before or during the shaping of the total mixture to give the resulting mouldings and takes place mainly or completely after the shaping.

2. Method according to Claim 1, **characterized in that** the method comprises the following steps in at least one zone of the method:
a) feeding in of a respective available starch;
b) action of a respective first plasticizer on the one available starch in each case;
c) transfer of the one available starch in each case into a respective first fluid, a respective first mixture being formed; and
d) transfer of a respective starch having network capabilities into a respective second fluid by the action of a respective second plasticizer;
e) transfer of the respective second fluid into a respective third fluid;
f) introduction of the respective second fluid from step d) and/or of the respective third fluid from step e) into one of the respective first mixtures from steps a) to c);
g) combination of the respective mixtures from steps a) to f) to give at least one molecular disperse total mixture;
h) forming of at least one film from the at least one total mixture formed in step g);
i) feeding of the at least one film formed in step h) into a shaping unit and production of resulting mouldings from the at least one film; in particular feeding of the at least one film formed in step h) into a continuous encapsulation unit, for example a rotary die unit, and production of welded soft capsules containing a filler or active substance;
j) initiation of the formation of a starch network from the at least one total mixture formed in step g), by homocrystallization between respective macromolecules of in each case at least one starch having network capabilities with one another and/or heterocrystallization between these respective macromolecules and respective macromolecules of in each case the at least one available starch after steps a) to h) or a) to i) have been implemented;
k) establishing of the desired plasticizer or water content of the resulting mouldings, the soft capsules, by conditioning with a time-based temperature and relative humidity curve provided.

3. Method according to either of Claims 1 and 2, **characterized in that,** in at least one of the steps d) to g), in particular after step f) and before step h), a plasticizer is at least partly actively removed from the method by evacuation techniques.

4. Method according to any of Claims 1 to 3, **characterized in that** the second fluid containing starch having network capabilities is superheated in step d) and the third fluid is supercooled in step e).

5. Method according to any of Claims 1 to 4, **characterized in that** a foreign nucleating agent is fed in at least once in at least one of the steps a) to g) and/or the second or third fluid is treated with ultrasound during or after step e).

6. Method according to any of the preceding Claims 1 to 5, **characterized in that** at least one additive is fed to the method in at least one of the steps a) to g).

7. Method according to any of the preceding Claims 1 to 6, **characterized in that,** in at least one of the steps a) to g), preferably in one of the steps a) to c), a special additive is added and the resulting starch gel having high toughness contains the special additive in the form of a highly disperse phase, the mean size of this phase being in the range 50 µm - 0.07 µm, preferably in the range 20 µm - 0.07 µm, more preferably in the range 7 µm - 0.07 µm, in particular in the range 3 µm - 0.07 µm, most preferably 1 µm - 0.07 µm.

8. Method according to any of the preceding Claims 1 to 7, **characterized in that** a film formed in step h) is divided in the longitudinal direction into two halves which are fed separately over the same distance and at the same speed to a rotary die unit so that the two halves of the welded soft capsules have experienced an identical history, i.e. were formed into a film simultaneously and in parallel in step h).

9. Method according to any of the preceding Claims 1 to 8, **characterized in that** steps a) to h) are carried out in parallel in two different zones of the method and the two halves of the welded soft capsules are produced by the rotary die method from two films which originate from the two different zones of the method.

10. Moulding in the form of a soft capsule, **characterized in that** the soft capsule has been produced by a method according to any of Claims 1 to 9.

11. Use of the soft capsules according to Claim 10 for the production of controlled release applications.

12. Multi layer moulding in the form of a multi-layer soft capsule, **characterized in that** at least one layer of the soft capsule consists of starch gel, which layer has been produced by a method according to any of Claims 1 to 9.

13. Soft capsule produced by a method according to Claims 1 to 9, **characterized in that** the soft capsule shell is in the form of a film of not more than 0.3 mm thickness and, after this film has been dried and is brought in this state into an atmosphere having a relative humidity of not more than 43% at room temperature and is stored there until the weight of the film no longer changed and the film has a water content of W₄₃ and after the film is then brought into an atmosphere having a relative humidity of at least 90% at room temperature until the weight no longer changes and the film has assumed a water content of W₉₀, the difference between the water contents W₉₀ - W₄₃ in percent by weight is 3% - 25%.

14. Soft capsule according to Claim 13, **characterized in that** the crystalline fraction of the soft capsule shell, obtained by separation of the crystalline fraction from the amorphous fraction of the wide-angle X-ray diffraction curve of a sample with 7 to 14% by weight of water, between the scattering angles 3° < 2 theta < 37°, is 15% - 100%.

15. Soft capsule according to either of Claims 13 and 14, **characterized in that** the soft capsule shell consists of single-phase, transparent starch gel, in particular has been produced by the rotary die method.

16. Soft capsule according to any of the preceding Claims 13 to 15, **characterized in that** the soft capsule shell contains a resistant starch in the form of an additive, in particular in a proportion in percent by weight of 1% - 70%.

17. Soft capsule according to any of the preceding Claims, **characterized in that** the soft capsule shell has a prebiotic action and/or has a plasticizer content of about 10% - 95% compared with a TPS soft capsule with comparable plasticizer content.

## Revendications

1. Procédé de fabrication d'objets moulés à base de gel d'amidon, qui ce **caractérise en ce que**
a. le gel d'amidon est formé à partir d'un mélange global d'au moins un amidon de base et d'au moins un amidon capable de se réticuler par homo et/ou hétérocristallisation dans lequel l'amidon capable de se réticuler présente un degré de ramification Q_{b} inférieur à 0.01,
b. et que les deux composants sont préparés séparément et individuellement, les amidons de base étant plastifiés et les amidons capables de se réticuler dissous ;
c. et que les composants sont mélangés en dispersion moléculaire dans cet état après leur réunion en un mélange global,
d. et que la formation de gel ou de réseau est engagée au moins partiellement avant ou pendant la transformation du mélange global pour former les objets moulés pour s'effectuer principalement ou entièrement après la transformation.

2. Procédé selon la revendication 1 **caractérisé en ce que** le procédé s'effectue selon les étapes suivantes dans au moins une zone de procédure :
a) Ajouter à chaque fois un amidon de base ;
b) Action chimique d'un premier plastifiant sur l'amidon de base,
c) Transférer un amidon de base dans un premier fluide, pour former un premier mélange ;
d) Transférer un amidon capable de se réticuler dans un deuxième fluide sous l'action d'un deuxième plastifiant ;
e) Transférer le deuxième fluide dans un troisième fluide ;
f) Introduire le deuxième fluide de l'étape d) et/ou du troisième fluide de l'étape e) dans l'un des premiers mélanges des étapes a) à c);
g) Réunir les mélanges des étapes a) à f) dans au moins un mélange global à dispersion moléculaire ;
h) Former au moins un film à partir d'au moins un mélange global réalisé à l'étape g) ;
i) Introduire au moins un film réalisé à l'étape h) dans un dispositif de transformation et fabrication d'objets moulés à partir d'au moins un film ; Introduire en particulier au moins un film réalisé à l'étape h) dans un dispositif d'encapsulation en continu, par exemple dans un dispositif de découpage rotatif (rotary-die), et réaliser des capsules molles soudées contenant une matière de charge ou des substances actives ;
j) Introduction de la formation d'un réseau d'amidon à partir au moins d'un mélange global réalisé à l'étape h), par homocristallisation entre les macromolécules d'un amidon capable de se réticuler et/ou par hétérocristallisation entre ces macromolécules et les macromolécules d'un amidon de base, une fois achevées les étapes a) à h) ou a) à i).
k) Réglage de la teneur du plastifiant désiré ou de l'eau concernant les objets moulés réalisés, les capsules molles, en conditionnant le déroulement prévu dans le temps de la température et de l'hygrométrie.

3. Procédé selon l'une des revendications 1 ou 2 **caractérisé en ce que** dans l'une au moins des étapes d) à g) en particulier après l'étape f) et avant l'étape h), un plastifiant pour le moins partiellement actif est éloigné de la procédure à l'aide de techniques d'évacuation.

4. Procédé selon l'une des revendications 1 ou 3 **caractérisé en ce que** lors de l'étape d) le deuxième fluide contenant l'amidon capable de se réticuler est surchauffé et que le troisième fluide est superréfrigéré à l'étape e).

5. Procédé selon l'une des revendications précédentes 1 à 4, **caractérisé en ce que** dans l'une au moins des étapes a) à g), il a été ajouté au moins une fois une agent de nucléation et/ou le deuxième ou le troisième fluide a été traité aux ultrasons pendant ou après l'étape e).

6. Procédé selon l'une des revendications précédentes 1 à 5, **caractérisé en ce que** au moins un additif est ajouté au procédé au cours de l'une au moins des étapes a) à g).

7. Procédé selon l'une des revendications précédentes 1 à 6, **caractérisé en ce que** pour le moins au cours de l'une des étapes a) à g), de préférence au cours de l'une des étapes a) à c) un additif spécial est ajouté et que le gel d'amidon à forte viscosité qui en résulte contient cet additif spécial sous forme d'une phase hautement disperse, où la taille moyenne de cette phase se situe entre 50µm - 0.07µm, de préférence entre 20µm - 0.07µm, encore mieux entre 7µm - 0.07µm, en particulier entre 3µm - 0.07µm, le mieux étant entre 1µm - 0.07µm,

8. Procédé selon l'une des revendications précédentes 1 à 7, **caractérisé en ce qu'un** film formé au cours de l'étape h) est partagé en deux moitiés dans le sens de la longueur, moitiés acheminées séparément sur un parcours égal et avec la même vitesse vers un dispositif de découpage rotatif (rotary-die) de telle sorte que les deux moitiés des capsules molles soudées subissent une préhistoire identique, c'est-à-dire soient moulées en un film en même temps et en parallèle au cours de l'étape h).

9. Procédé selon l'une des revendications précédentes 1 à 8, **caractérisé en ce que** les étapes a) à h) sont exécutées de manière parallèle dans deux différentes zones de procédure et que les deux moitiés des capsules molles soudées sont réalisées selon le procédé de découpage rotatif (rotary-die) à partir de deux films provenant de deux différentes zones de procédure.

10. Objet moulé en forme de capsule molle, **caractérisée en ce que** la capsule molle a été réalisée par un procédé répondant à l'une des revendications 1 à 9.

11. Utilisation de la capsule molle selon la revendication 10 pour réaliser des applications de retard contrôlé (controlled release).

12. Objets moulés à plusieurs couches sous forme de capsules molles à plusieurs couches, **caractérisées en ce qu'une** couche au moins des capsules molles est un gel d'amidon et qu'elles sont réalisées selon l'une des revendications 1 à 9.

13. Capsules molles fabriquées par un procédé répondant aux revendications 1 à 9, **caractérisées en ce que** l'enveloppe de la capsule molle formant un film épais de 0.3mm, que, une fois ce film séché et mis dans cet état dans une atmosphère comportant au maximum 43% d'humidité à la température ambiante, qu'il y soit conservé ainsi aussi longtemps jusqu'à ce que le poids du film ne change plus et que le film présente une teneur en eau de W₄₃ et que, une fois que le film aura été mis ensuite dans une atmosphère avec au moins 90% d'humidité à la température ambiante jusqu'à ce que son poids ne change plus et qu'il ait pris une teneur en eau de W₉₀, la différence des teneurs en eau W₉₀ - W₉₀ se situe en poids % entre 3% - 25%.

14. Capsule molle selon la revendication 13, **caractérisée en ce que** la part cristalline de l'enveloppe de la capsule molle, obtenue par séparation de la part cristalline de la part amorphe de la courbe de diffraction radiographique de grand angulaire d'une épreuve (échantillon) avec un poids % en eau de 7 à 14, se situe entre les angles de dispersion 3° < 2δ < 37°, 15% - 100%.

15. Capsule molle selon la revendication 13 ou 14, **caractérisée en ce que** l'enveloppe de la capsule molle est réalisée à partir de gels d'amidon transparents, monophasés, obtenue en particulier à partir de la procédure de découpage rotatif (rotary-die).

16. Capsule molle selon la revendication 13 à 15, **caractérisée en ce que** l'enveloppe de la capsule molle contient un amidon résistant sous forme d'une substance additive, en particulier avec une part en poids % de 1% - 70%.

17. Capsule molle selon l'une des revendications précitées, **caractérisée en ce que** l'enveloppe de la capsule molle dispose d'un effet prébiotique et/ou présente par rapport à une capsule TPS avec une teneur en plastifiant comparable, un indice glycémique réduit de 10% - 95%, de préférence de 20% - 95%, le mieux étant de 30% - 95%.
